# EUROPEAN PATENT SPECIFICATION

(11) **EP 4 548 843 B1**
(45) Date of publication and mention of the grant of the patent: **11.11.2026**
(21) Application number: 23831892.7
(22) Date of filing: 28.06.2023
(51) Int. Cl.: A61B 5/145, A61B 5/00, A61B 5/332, A61B 5/0537, A61B 5/1455, A61B 5/256, A61B 5/263

(54) **ELECTRONIC DEVICE COMPRISING OPTICAL SENSOR AND BIO-IMPEDANCE DETECTION SENSOR**
ELEKTRONISCHE VORRICHTUNG MIT OPTISCHEM SENSOR UND BIOIMPEDANZDETEKTIONSSENSOR
DISPOSITIF ÉLECTRONIQUE COMPRENANT UN CAPTEUR OPTIQUE ET UN CAPTEUR DE DÉTECTION DE BIO-IMPÉDANCE

(30) Priority: 30.06.2022 KR 20220080327
(43) Date of publication of application: 07.05.2025
(73) Proprietor: Samsung Electronics Co., Ltd., Suwon-si, Gyeonggi-do 16677 (KR)
(72) Inventor: KWON, Yongjoo, Suwon-si Gyeonggi-do 16677 (KR); SEO, Minwoo, Suwon-si Gyeonggi-do 16677 (KR); CHO, Jeongho, Suwon-si Gyeonggi-do 16677 (KR); CHAE, Sungwon, Suwon-si Gyeonggi-do 16677 (KR)
(74) Representative: HGF
(86) International application number: PCT/KR2023/009008
(87) International publication number: WO 2024/005533

(56) References cited:
- EP-A1- 3 281 577
- WO-A1-2021/096216
- KR-A- 20190 081 051
- KR-A- 20200 012 598
- KR-A- 20200 012 598
- KR-A- 20200 119 501
- US-A1- 2010 004 517
- US-A1- 2014 081 093
- US-A1- 2014 350 413
- US-A1- 2017 340 209
- US-A1- 2019 059 821
- US-A1- 2019 200 883
- US-A1- 2019 246 977
- US-A1- 2022 190 188
- YUJIA ZHANG ET AL: "A Bioinspired Wireless Epidermal Photoreceptor for Artificial Skin Vision", ADVANCED FUNCTIONAL MATERIALS, WILEY, HOBOKEN, USA, vol. 30, no. 22, 9 April 2020 (2020-04-09), pages n/a, XP072412774, ISSN: 1616-301X, DOI: 10.1002/ADFM.202000381

## Description

### [Technical Field]

The disclosure relates to an electronic device including an optical sensor and a bio-impedance detection sensor.

### [Background Art]

With the development of information and communication technology and semiconductor technology, various functions are packed in one portable electronic device. For example, an electronic device may implement not only communication functions but also entertainment functions, such as playing games, multimedia functions, such as playing music and videos, communication and security functions for mobile banking, and scheduling and e-wallet functions. These electronic devices have been downsized to be conveniently carried by users.

KR 2020 0012598 A relates to a device and method for measuring biosignals.

US 2019200883A1 relates to a bio-signal measuring apparatus.

KR 2020 0119501 A relates to estimating bio-information.

US 2017340209 A1 relates to blood pressure monitoring using a multi-function wrist-worn device.

### [Detailed Description of the Invention]

### [Technical Problem]

If the body's electrolyte concentration is outside the designated range, disease (e.g., kidney disease, renal failure, diabetes insipidus) or symptoms (e.g., dehydration, diarrhea, vomiting) may occur. The electrolyte concentration may be detected by measuring the electrical potential of the blood. However, when determining electrolyte concentration through blood sampling, continuous monitoring is not possible and user convenience may be low.

A wearable electronic device may remain in contact with the user's body for a long time and may thus be useful for medical or healthcare purposes. For example, an electronic device may detect the user's biometric information from a sensor equipped therein. The detected biometric information may be stored in the electronic device or transmitted to medical institutions to be used for user healthcare purposes.

According to various embodiments of the disclosure, there may be provided an electronic device for non-invasively detecting electrolyte concentration using an optical sensor and a bio-impedance detection sensor.

According to various embodiments of the disclosure, there may be provided an electronic device that is worn by the user to continuously detect the user's electrolyte concentration.

The disclosure is not limited to the foregoing embodiments but various modifications or changes may rather be made thereto.

### [Technical Solution]

According to an aspect of the present disclosure, an electronic device is provided as defined in the appended claims. The extent of protection conferred shall be determined from the claims.

### [Advantageous Effects]

According to various embodiments of the disclosure, an electronic device may determine an electrolyte concentration of an object based on the intensity of light detected by an optical sensor and the magnitude of impedance detected by a bio-impedance detection sensor. As the electrolyte concentration is determined non-invasively using light and current, user convenience may be increased. Further, since electrolyte concentration is constantly determined, healthcare services related to electrolyte concentration (e.g., thirst notification) may be provided.

Other various effects may be provided directly or indirectly in the disclosure.

### [Brief Description of Drawings]

The above and other aspects, features, and advantages of certain embodiments of the present disclosure will be more apparent from the following description taken in conjunction with the accompanying drawings, in which:
FIG. 1 is a block diagram illustrating an electronic device in a network environment according to various embodiments of the disclosure;
FIG. 2 is a front perspective view illustrating an electronic device according to various embodiments of the disclosure;
FIG. 3 is a rear perspective view illustrating the electronic device of FIG. 2;
FIG. 4 is an exploded perspective view illustrating an electronic device according to various embodiments of the disclosure;
FIG. 5 is a view illustrating an electronic device including an optical sensor and a bio-impedance detection sensor according to various embodiments of the disclosure;
FIG. 6 is a block diagram illustrating an electronic device including an optical sensor and a bio-impedance detection sensor according to various embodiments of the disclosure;
FIG. 7 is a perspective view illustrating a rear plate where a bio-impedance detection sensor is mounted according to various embodiments of the disclosure; and
FIGS. 8, 9, 10, 11, and 12 are views illustrating a placement structure of an electronic device including an optical sensor and a bio-impedance detection sensor according to various embodiments of the disclosure.

### [Mode for Carrying out the Invention]

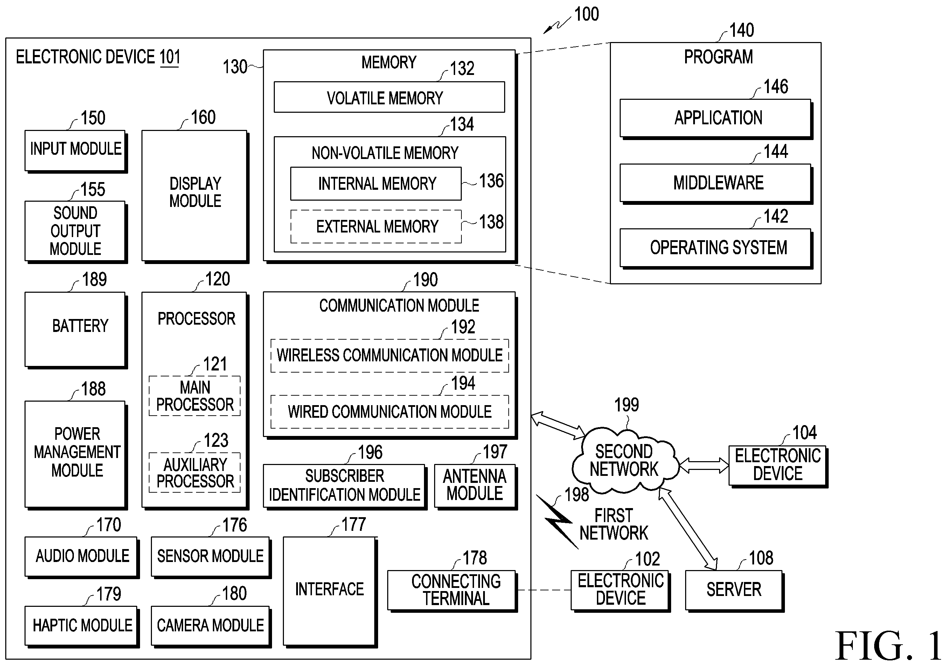

FIG. 1 is a block diagram illustrating an electronic device in a network environment according to various embodiments of the disclosure;

Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to various embodiments, the electronic device 101 may communicate with the electronic device 104 via the server 108. According to various embodiments, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In various embodiments, at least one (e.g., the connecting terminal 178) of the components may be omitted from the electronic device 101, or one or more other components may be added in the electronic device 101. According to various embodiments, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to various embodiments, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to various embodiments, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to various embodiments, the auxiliary processor 123 (e.g., an image signal processor or a communication processor) may be implemented as part of another component (e.g., the camera module 180 or the communication module 190) functionally related to the auxiliary processor 123. According to various embodiments, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to various embodiments, the receiver may be implemented as separate from, or as part of the speaker.

The display module 160 may visually provide information to the outside (e.g., a user) of the electronic device 101. The display 160 may include, for example, a display, a hologram device, or a projector and control circuitry to control a corresponding one of the display, hologram device, and projector. According to various embodiments, the display 160 may include a touch sensor configured to detect a touch, or a pressure sensor configured to measure the intensity of a force generated by the touch.

The audio module 170 may convert a sound into an electrical signal and vice versa. According to various embodiments, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to various embodiments, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to various embodiments, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to various embodiments, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to various embodiments, the haptic module 179 may include, for example, a motor, a piezoelectric element, or an electric stimulator.

The camera module 180 may capture a still image or moving images. According to various embodiments, the camera module 180 may include one or more lenses, image sensors, image signal processors, or flashes.

The power management module 188 may manage power supplied to the electronic device 101. According to various embodiments, the power management module 188 may be implemented as at least part of, for example, a power management integrated circuit (PMIC).

The battery 189 may supply power to at least one component of the electronic device 101. According to various embodiments, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to various embodiments, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to various embodiments, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to various embodiments, the antenna module may include an antenna including a radiator formed of a conductor or conductive pattern formed on a substrate (e.g., a printed circuit board (PCB)). According to various embodiments, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to various embodiments, other parts (e.g., radio frequency integrated circuit (RFIC)) than the radiator may be further formed as part of the antenna module 197.

According to various embodiments, the antenna module 197 may form a mmWave antenna module. According to various embodiments, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to various embodiments, instructions or data may be transmitted or received between the electronic device 101 and the external electronic device 104 via the server 108 coupled with the second network 199. The external electronic devices 102 or 104 each may be a device of the same or a different type from the electronic device 101. According to various embodiments, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an Internet-of-things (IoT) device. The server 108 may be an intelligent server using machine learning and/or a neural network. According to various embodiments, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments of the disclosure may be one of various types of electronic devices. The electronic devices may include, for example, a portable communication device (e.g., a smartphone), a computer device, a portable multimedia device, a portable medical device, a camera, a wearable device, or a home appliance. According to various embodiments of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to various embodiments, the module may be implemented in a form of an application-specific integrated circuit (ASIC).

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

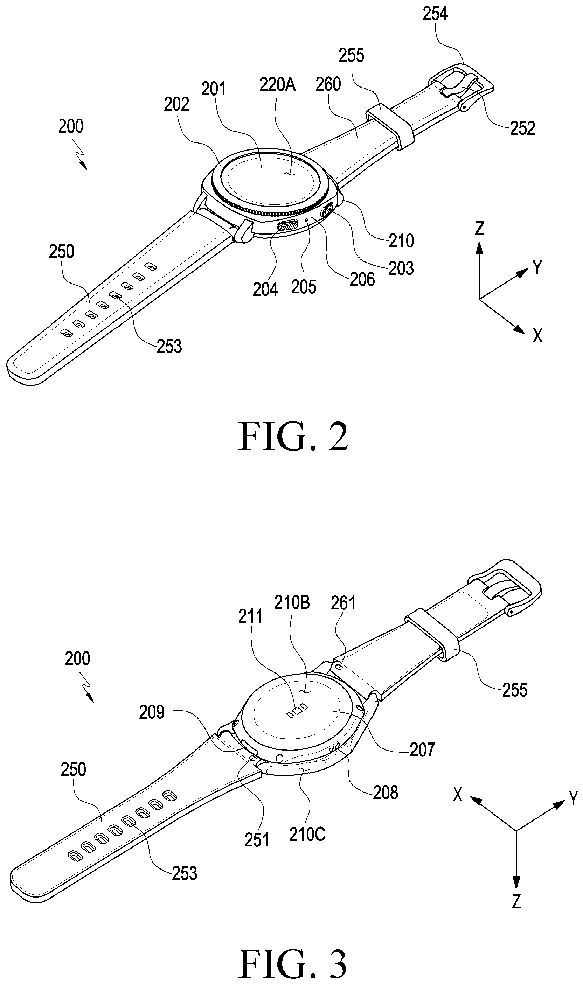

FIG. 2 is a front perspective view illustrating an electronic device according to various embodiments of the disclosure; FIG. 3 is a rear perspective view illustrating the electronic device of FIG. 2;

Referring to FIGS. 2 and 3, an electronic device 200 is a watch-type electronic device and may be worn by the user. For example, the electronic device 200 may be a smart watch or a wearable electronic device that may be worn on the user's wrist.

The electronic device according to various embodiments may be one of various types of devices. For example, the electronic device may be the smart watch of FIGS. 2 to 4. The electronic device of the disclosure is not limited to the smart watch illustrated in FIGS. 2 to 4. For example, the electronic device may be earbuds and/or a smart watch. As another example, an electronic device may be manufactured as a module and attached to another electronic device (e.g., exercise equipment). According to various embodiments, the electronic device of the disclosure may be an earring wearable on an ear. According to various embodiments, the electronic device of the disclosure may be a smart ring wearable on a finger.

An electronic device 200 according to various embodiments may include a housing 210 including a first surface (or front surface) 210A, a second surface (or rear surface) 210B, and a side surface 210C surrounding the space between the first surface 210A and the second surface 210B and attachment members 250 and 260 connected to at least part of the housing 210 and configured to allow the electronic device 200 to be removably worn on the user's body portion (e.g., the user's wrist or ankle).

In another embodiment (not shown), the housing may denote a structure forming part of the first surface 210A, the second surface 210B, and the side surfaces 210C. According to various embodiments, at least part of the first surface 210A may have a substantially transparent front plate 201 (e.g., a glass plate or polymer plate including various coat layers). The second surface 210B may be formed by a rear plate 207 that is substantially opaque. The rear plate 207 may be formed of, e.g., laminated or colored glass, ceramic, polymer, metal (e.g., aluminum, stainless steel (STS), or magnesium), or a combination of at least two thereof. The side surface 210C may be formed by a side bezel structure (or a "side member") 206 that couples to the front plate 201 and the rear plate 207 and includes a metal and/or polymer. According to various embodiments, the rear plate 207 and the side bezel plate 206 may be integrally formed together and include the same material (e.g., a metal, such as aluminum). The attachment members 250 and 260 may be formed of various materials in various shapes. A uni-body structure or multiple unit links which is flexible may be formed of fabric, leather, rubber, urethane, metal, ceramic, or a combination of at least two thereof.

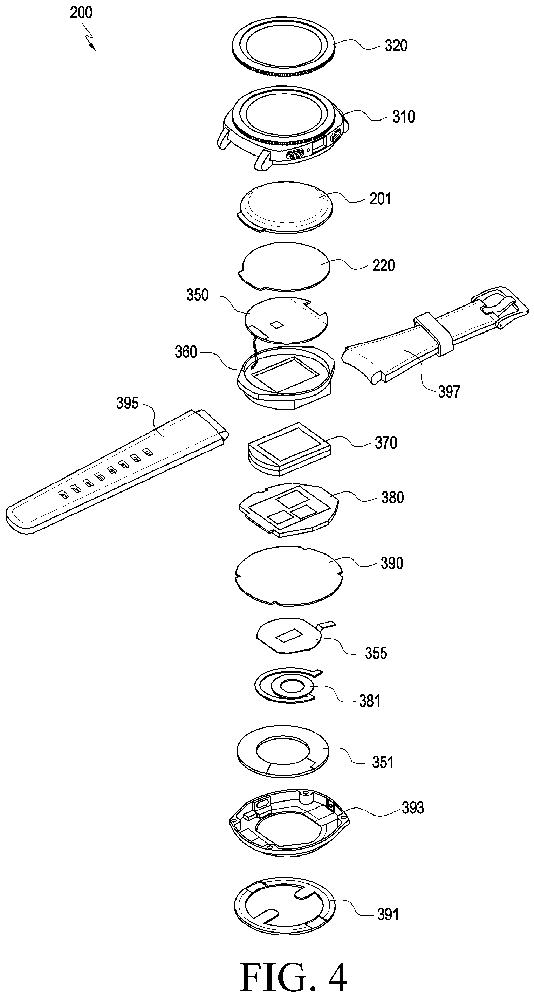

According to various embodiments, the electronic device 200 may include at least one or more of a display (e.g., the display 220 of FIG. 4), audio modules 205 and 208, a sensor module 211, key input devices 202, 203, and 204, and a connector hole 209. According to various embodiments, the electronic device 200 may exclude at least one (e.g., the key input devices 202, 203, and 204, connector hole 209, or sensor module 211) of the components or may add other components.

The display 220 may be exposed through a significant portion of the front plate 201. The display 220 may have a shape corresponding to the shape of the front plate 201, e.g., a circle, ellipse, or polygon. The display 220 may be coupled with, or disposed adjacent, a touch detection circuit, a pressure sensor capable of measuring the strength (pressure) of touches, and/or fingerprint sensor.

The audio modules 205 and 208 may include a microphone hole 205 and a speaker hole 208. The microphone hole 205 may have a microphone inside to obtain external sounds. According to various embodiments, there may be a plurality of microphones to be able to detect the direction of a sound. The speaker hole 208 may be used for an external speaker or a receiver for phone talks. According to various embodiments, the speaker hole 208 and the microphone hole 205 may be implemented as a single hole, or speaker may be included without the speaker hole 208 (e.g., piezo speaker).

The sensor module 211 may produce an electrical signal or data value corresponding to the internal operation state or external environment state of the electronic device 200. The sensor module 211 may include, e.g., a biometric sensor module 211 (e.g., a heartrate monitor (HRM) sensor) disposed on the second surface 210B of the housing 210. The electronic device 200 may include a sensor module not shown, e.g., at least one of a gesture sensor, a gyro sensor, a barometric sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include a wheel key 202 disposed on the first surface 210A of the housing 210 to be rotatable in at least one direction and/or key button-type key input devices 203 and 204 disposed on the side surface 210C of the housing 210. The wheel key may have a shape corresponding to the shape of the front plate 201. According to another embodiment, the electronic device 200 may exclude all or some of the above-mentioned key input devices 202, 203, and 204, and the excluded key input devices 202, 203, and 204 may be implemented in other forms, e.g., as soft keys on the display 220. The connector hole 209 may receive a connector (e.g., a universal serial bus (USB) connector) for transmitting and receiving power and/or data to/from an external electronic device. Another connector hole may be included for receiving a connector for transmitting and receiving audio signals to/from the external electronic device. The electronic device 200 may further include a connector cover to cover at least part of, e.g., the connector hole 209 and preventing undesirable materials from entering the connector hole.

The attachment members 250 and 260 may detachably be bound to at least portions of the housing 210 via locking members 251 and 261. The attachment members 250 and 260 may include one or more of a fixing member 252, fixing member coupling holes 253, a band guide member 254, and a band fixing ring 255.

The fixing member 252 may be configured to allow the housing 210 and the attachment members 250 and 260 to be fixed to the user's body portion (e.g., wrist or ankle). The fixing member coupling holes 253 may fix the housing 210 and the attachment members 250 and 260 to the user's body portion, corresponding to the fixing member 252. The band guide member 254 may be configured to restrict movement of the fixing member 252 to a certain range when the fixing member 252 fits into one of the fixing member coupling holes 253, thereby allowing the attachment members 250 and 260 to be tightly bound onto the user's body portion. The band fixing ring 255 may limit the range of movement of the attachment members 250 and 260, with the attachment member 252 fitted into one of the attachment member coupling holes 253.

FIG. 4 is an exploded perspective view illustrating an electronic device according to various embodiments of the disclosure.

Referring to FIG. 4, the electronic device 200 may include a side bezel structure 310, a wheel key 320, a front plate 201, a display 220, a first antenna 350, a second antenna 351, a sensor module 355, a supporting member 360 (e.g., a bracket), a battery 370, a printed circuit board 380 (e.g., a first printed circuit board), an auxiliary printed circuit board 381 (e.g., a second printed circuit board), a sealing member 390, a rear plate 393, a rear cover 391, and attachment members 395 and 397. At least one of the components of the electronic device 200 may be the same or similar to at least one of the components of the electronic device 200 of FIG. 1 or 2 and no duplicate description is made below. The supporting member 360 may be disposed inside the electronic device 200 to be connected with the side bezel structure 310 or integrated with the side bezel structure 310. The supporting member 360 may be formed of, e.g., a metal and/or non-metallic material (e.g., polymer). The display 220 may be joined onto one surface of the supporting member 360, and the printed circuit board 380 may be joined onto the opposite surface of the supporting member 274. A processor, memory, and/or interface may be mounted on the printed circuit board 380 and/or the auxiliary printed circuit board 381. The processor may include one or more of, e.g., a central processing unit, an application processor, a graphic processing unit (GPU), a sensor processor, or a communication processor.

According to various embodiments, the memory may include, e.g., a volatile or non-volatile memory. The interface may include, e.g., a high definition multimedia interface (HDMI), a universal serial bus (USB) interface, a secure digital (SD) card interface, and/or an audio interface. The interface may electrically or physically connect, e.g., the electronic device 200 with an external electronic device and may include a USB connector, an SD card/multimedia card (MMC) connector, or an audio connector.

According to various embodiments, the side bezel structure 310 may function as an antenna of the electronic device 200. For example, a communication module (e.g., communication module 190 of FIG. 1) may transmit a wireless signal to the outside or receive a wireless signal from the outside using the side bezel structure 310. According to various embodiments, the side bezel structure 310 may be electrically connected to the communication module 190 positioned on the circuit board 380. According to various embodiments, the configuration of the side bezel structure 310 may be identical in whole or part to the configuration of the antenna module (e.g., the antenna module 197 of FIG. 1).

According to various embodiments, the battery 370 may be a device for supplying power to at least one component of the electronic device 200. The battery 189 may include, e.g., a primary cell which is not rechargeable, a secondary cell which is rechargeable, or a fuel cell. At least a portion of the battery 370 may be disposed on substantially the same plane as the printed circuit board 380. The battery 370 may be integrally or detachably disposed inside the electronic device 200.

According to various embodiments, the first antenna 350 may be disposed between the display 220 and the supporting member 360. The first antenna 350 may include, e.g., a near-field communication (NFC) antenna, a wireless charging antenna, and/or a magnetic secure transmission (MST) antenna. In various embodiments (e.g., FIG. 4), the first antenna 350 may be an NFC antenna. The first antenna 350 may perform short-range communication with an external device, wirelessly transmit/receive power necessary for charging, or transmit magnetic-based signals including payment data or short-range communication signals. According to various embodiments of the present invention, an antenna structure may be formed by a portion or combination of the side bezel structure 310 and/or the supporting member 360.

According to various embodiments, the second antenna 351 may be disposed between the circuit board 380 and the rear plate 393. The second antenna 351 may include, e.g., a near-field communication (NFC) antenna, a wireless charging antenna, and/or a magnetic secure transmission (MST) antenna. In various embodiments (e.g., FIG. 4), the second antenna 351 may be a wireless charging antenna. The second antenna 351 may perform short-range communication with an external device, wirelessly transmit/receive power necessary for charging, or transmit magnetic-based signals including payment data or short-range communication signals. According to another embodiment, an antenna structure may be formed of a portion or combination of the side bezel structure 310 and/or the rear plate 393. According to various embodiments, the sealing member 390 may be positioned between the side bezel structure 310 and the rear plate 393. The sealing member 390 may be configured to block moisture or foreign bodies that may enter the space surrounded by the side bezel structure 310 and the rear plate 393, from the outside.

According to various embodiments, the rear cover 391 may be positioned under the rear plate 393. At least a portion of the rear cover 391 may be exposed to the outside of the electronic device 200. The rear cover 391 may cover at least a portion of the sensor module 355 and/or the auxiliary printed circuit board 381.

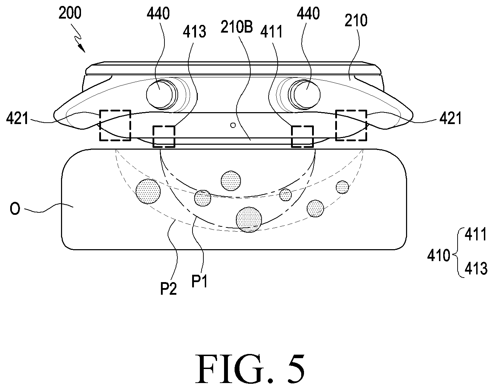

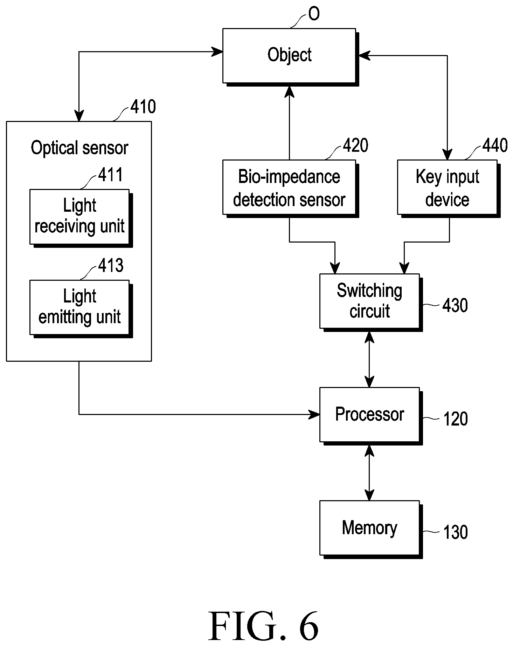

FIG. 5 is a view illustrating an electronic device including an optical sensor and a bio-impedance detection sensor according to various embodiments of the disclosure. FIG. 6 is a block diagram illustrating an electronic device including an optical sensor and a bio-impedance detection sensor according to various embodiments of the disclosure.

Referring to FIG. 5 and/or FIG. 6, the electronic device 200 may include a processor 120, a housing 210, an optical sensor 410, and a bio-impedance detection sensor 420. The configuration of the housing 210, the key input devices 203 and 204, and the bio-impedance detection sensor 420 of FIG. 5 may be identical in whole or part to the configuration of the housing 210, the key input devices 203 and 204, and the sensor module 211 of FIG. 2. The configuration of the processor 120 of FIG. 6 may be identical in whole or part to the configuration of the processor 120 of FIG. 1 . According to various embodiments, the bio-impedance detection sensor 420 may be referred to as a biosignal detection sensor.

According to various embodiments, the optical sensor 410 may detect an optical signal. For example, the optical sensor 410 may be a photoplethysmo graph (PPG) sensor for detecting the user's plethysmogram (PTG). For example, the optical sensor 410 may detect the intensity of light reflected from a portion of the object O. For example, the optical sensor 410 may include a light receiving unit 411 and a light emitting unit 413 spaced apart from the light receiving unit 411. According to various embodiments, the light emitting unit 413 may be a light source configured to emit light having a wavelength of 1450 nm to 2000 nm. For example, the light emitting unit 413 may emit light having a wavelength of 1400 nm to 1500 nm or 1900 nm to 2000 nm. According to various embodiments, the light receiving unit 411 may be a photodetector capable of detecting light having a wavelength of 1450 nm to 2000 nm. For example, the light receiving unit 411 may receive light having a wavelength of 1400 nm to 1500 nm or 1900 nm to 2000 nm. Each described range may include the endpoints within the range. According to various embodiments, at least a portion of the light emitted from the light emitting unit 413 may be transferred to the light receiving unit 411 through an optical path P1. According to various embodiments, when the electronic device 200 operates, the optical path P1 may be positioned in the object O. For example, the light radiated by the optical sensor 410 may use the body as a medium.

According to various embodiments, the optical sensor 410 may be disposed on the housing 210. For example, the optical sensor 410 may be disposed to face the rear surface 200B of the electronic device 200.

According to various embodiments, the bio-impedance detection sensor 420 may detect or sense a current or impedance. For example, the bio-impedance detection sensor 420 may detect biometric information such as bioelectric impedance analysis (BIA) information. For example, the bio-impedance detection sensor 420 may include a plurality of electrodes 421.

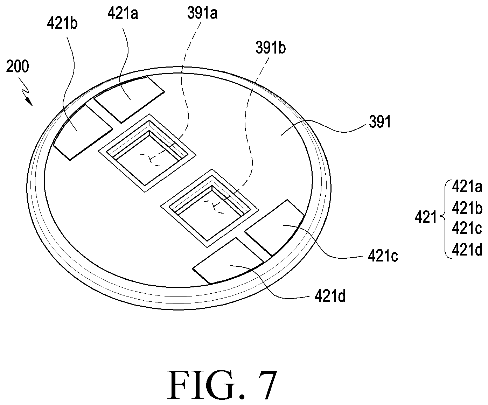

According to various embodiments, some of the plurality of electrodes 421 may transmit a current, and others of the plurality of electrodes 421 may receive a current. For example, some of the plurality of electrodes 412 may be referred to as a current transmitting unit, and others may be referred to as a current receiving unit. Referring to FIG. 7 together with FIGS. 5 and 6, the plurality of electrodes 421 may include a first electrode 421a, a second electrode 421b, a third electrode 421c, and a fourth electrode 421d spaced apart from each other. Some of the first electrode 421a, the second electrode 421b, the third electrode 421c, and the fourth electrode 421d may transmit a current, and others may receive a current. According to various embodiments, the electrode transmitting the current and the electrode receiving the current among the plurality of electrodes 421 may be changed based on a designated time interval and/or a designated rule. According to various embodiments, the plurality of electrodes 421 may transmit and receive current through the current path P2.

According to various embodiments, the plurality of electrodes 421 of the bio-impedance detection sensor 420 may transmit and receive the current as alternating current. The frequency of the current generated by the bio-impedance detection sensor 420 may be 20 kHz or more. For example, the frequency of the bio-impedance detection sensor 420 may be 20 kHz to 200 kHz.

According to various embodiments, the bio-impedance detection sensor 420 may be disposed on the housing 210. For example, the bio-impedance detection sensor 420 may be disposed to face the rear surface 200B of the electronic device 200.

According to various embodiments, at least a portion of the current path P2 through which the current of the bio-impedance detection sensor 420 passes may overlap at least a portion of the optical path P1 through which the light of the optical sensor 410 passes. For example, at least a portion of the optical path P1 may be positioned in the same space as at least a portion of the current path P2. According to various embodiments, 50% or more of the optical path P1 may be positioned in the same space as the current path P2.

According to various embodiments, the processor 120 may determine the intensity I of light using the optical sensor 410. For example, the processor 120 may determine the intensity of light based on the optical signal sensed by the optical sensor 410.

According to various embodiments, the processor 120 may determine the local impedance Z of the object O using the bio-impedance detection sensor 420. For example, the processor 120 may determine the local impedance of the object O based on the current and/or the impedance (e.g., the magnitude of the impedance) detected by the bio-impedance detection sensor 420.

The processor 120 determines the biometric component of the object O based on the information obtained from the optical sensor 410 and the biometric impedance detection sensor 420. The biometric component includes an electrolyte concentration. The object O may be the body of the user or the body of the subject. For example, the object O may be the wrist or earlobe of the human body. According to various embodiments, the electrolyte may include sodium ions (Na+), potassium ions (K+), chlorine ions (Cl-), and/or bicarbonate ions (HCO₃-). According to various embodiments, by determining the electrolyte concentration, the processor 120 may determine a disease that may occur due to an imbalance in the electrolyte concentration. According to various embodiments, the electronic device 200 may output the electrolyte concentration e to the user using a display (e.g., the display module 160 of FIG. 1). According to various embodiments, when the detected electrolyte concentration e of the object O is outside a designated range, the processor 120 may suggest an action (e.g., water or electrolyte intake) necessary for the user. According to various embodiments, the accuracy of measuring the body moisture content of the object O may be increased based on the local impedance. For example, when the impedance of the whole body or the upper body is detected, the electrolyte concentration assumed to be a constant concentration may be calculated considering the electrolyte concentration e determined using the electronic device 200 of the disclosure.

According to the invention, the electrolyte concentration e meets Equation 1 below.
[Equation 1] $electrolyte concentration \left(e\right) = \frac{ε {\left(nd\right)}^{2}}{kZln \left(\frac{{I}_{0}}{I}\right)}$

In Equation 1, ε is the light absorption coefficient of the electrolyte in the blood (e.g., in the blood of the user), d is the distance between the current transmitting unit and the current receiving unit at the plurality of electrodes 421, Z is the magnitude of the impedance measured by the bio-impedance detection sensor 420, I₀ is the intensity of light emitted from the light emitting unit 413, and I is the intensity of light incident on the light receiving unit 411.

In Equation 1, n may be a proportional constant between the length L of the current path P2 and the electrode distance d. For example, n may be determined based on Equation 2 below.
[Equation 2] $L = nd$

In Equation 1, k may be a proportional constant between the conductivity σ of the electrolyte and the electrolyte concentration e. For example, k may be determined based on Equation 3 below.
[Equation 3] $σ = ke$ According to various embodiments, the current path P2 may be schematically shown in a cylindrical shape. For example, the local impedance Z may be expressed by Equation 4 below.
[Equation 4] $Z = \frac{{L}^{2}}{σ V}$

In Equation 4, L may be the length of the current path P2 through which the current flows. L may be determined based on the distance d between the plurality of electrodes 421. σ is the conductivity. σ may be determined based on the electrolyte concentration e. According to various embodiments, the length L of the current path P2 may be proportional to the distance d between the plurality of electrodes 421. The conductivity σ may be proportional to the electrolyte concentration e. For example, the length L of the current path P2 may meet Equation 2 above, and the conductivity σ may meet Equation 3 above.

Referring to Equation 2, Equation 3, and Equation 4, the electrolyte concentration e may be derived by Equation 5 below. According to various embodiments, when the length L of the current path P2 and the distance d between the plurality of electrodes 421 are in a simple proportional relationship (e.g., Equation 2), and the conductivity σ and the electrolyte concentration e are in a simple proportional relationship (e.g., Equation 3), the electrolyte concentration e may be expressed as Equation 5 below.
[Equation 5] $electrolyte concentration \left(e\right) = \frac{{\left(nd\right)}^{{}^{2}}}{kZV}$

According to various embodiments, the incident light and the transmitted light may meet Equation 6 below. For example, when the optical path P1 is substantially cylindrical, the intensity I of the light detected or sensed by the light receiving unit 411 of the optical sensor 410 and the intensity I₀ of the light radiated from the light emitting unit 413 of the optical sensor 410 may meet Equation 6 below. In Equation 6, ε means the light absorption coefficient of the light absorbing material, C means the distribution of the light absorbing material, and V_{P} means the light path volume.

[Equation 6] $\frac{I}{{I}_{0}} = exp \left(-ε{CV}_{p}\right)$

Referring to Equation 6, the volume CV_{P} of the electrolyte may be referred to as Equation 7 below. For example, the volume V of the current path P2 may be substantially the same as the electrolyte volume CV_{P} below.
[Equation 7] $electrolyte volume \left({CV}_{p}\right) = - ln \left(\frac{I}{{I}_{0}}\right) \frac{I}{ε}$

According to various embodiments, the electrolyte concentration e may be derived from Equation 1 above using Equation 5 and Equation 7.

According to various embodiments, the memory 130 may store a coefficient and/or a proportional constant for determining the electrolyte concentration e. For example, the memory 130 may store the light absorption coefficient ε of the electrolyte in the blood, the proportional constant n between the length L of the current path P2 and the electrode distance d, and/or the proportional constant k between the conductivity σ of the electrolyte and the electrolyte concentration e. The light absorption coefficient ε of the electrolyte in the blood, the proportional constant n between the length L of the current path P2 and the electrode distance d, and/or the conductivity σ of the electrolyte may be stored in the memory 130 as a designated value. According to various embodiments, the processor 120 may determine the electrolyte concentration e by reflecting the light absorption coefficient ε of the electrolyte in blood, the proportional constant n between the length L of the current path P2 and the electrode distance d, and/or the conductivity σ of the electrolyte stored in the memory 130. According to various embodiments, the electronic device 200 may include determining the intensity of light reflected from a portion of the object O using the optical sensor 410, determining the magnitude of the impedance of a portion of the object O using the bio-impedance detection sensor 420, and determining the electrolyte concentration e of the object O based on the intensity of the light and the magnitude of the impedance.

According to various embodiments, the memory 130 may store the intensity I₀ of light emitted from the light emitting unit 413. The intensity I₀ of the light emitted from the light emitting unit 413 may be changed according to the type of the light emitting unit 413 used in the electronic device 200.

The electrolyte concentration e is determined using machine learning. An electrolyte concentration (e) prediction model is trained using data obtained using data obtained by applying the electronic device 200 including the optical sensor 410 and the bio-impedance detection sensor 420 to a plurality of subjects. The electrolyte concentration of a new user may be detected using the trained prediction model. According to various embodiments, an electrolyte concentration prediction model may be implemented using a multilinear regression model or a nonlinear regression model (e.g., DNN, CNN, or RNN). The light absorption coefficient ε of the electrolyte in the blood, the proportional constant n between the length L of the current path P2 and the electrode distance d, and the conductivity σ of the electrolyte are learned using machine learning. The light absorption coefficient ε of the electrolyte in the blood, the proportional constant n between the length L of the current path P2 and the electrode distance d, and the conductivity σ of the electrolyte are determined using machine learning reflecting a plurality of clinical data and/or data of various users.

According to various embodiments, electrolyte concentration data of a plurality of subjects may be obtained using the electronic device 200. Further, the electrolyte concentration data of the plurality of subjects may be obtained through blood collection. Using machine learning, a model of electrolyte concentration data obtained using the electronic device 200 of the disclosure and electrolyte concentration data obtained through blood collection may be designed. The electronic device 200 may determine the electrolyte concentration by reflecting the designed model.

According to various embodiments, the electronic device 200 may include a switching circuit 430. According to various embodiments, the switching circuit 430 may selectively and electrically connect the key input devices 203 and 204 or the bio-impedance detection sensor 420 to the processor 120. According to various embodiments, in a state in which the user wears the electronic device 200 on the left wrist and the user brings his body (e.g., a right finger) in contact to the key input devices 203 and 204, the processor 120 may determine the electrolyte concentration of the user using the optical sensor 410 and the bio-impedance detection sensor 420 for a predetermined time, and may determine biometric information (e.g., an electrocardiogram (ECG) or body composition) of the user using the key input device 440 for another time. For example, when the electronic device 200 detects the electrolyte concentration, the switching circuit 430 may connect the bio-impedance detection sensor 420 to the processor 120. When the electronic device 200 detects the electrocardiogram and/or the body composition, the switching circuit 430 may connect at least one (e.g., the first electrode 421a, the second electrode 421b, the third electrode 421c, and/or the fourth electrode 421d of FIG. 7) of the plurality of bio-impedance detection sensors 420 and the key input device 440 to the processor 120.

FIG. 7 is a perspective view illustrating a rear cover where a bio-impedance detection sensor is mounted according to various embodiments of the disclosure.

Referring to FIG. 7, the electronic device 200 may include a plurality of electrodes 321 and a rear cover 391. The configuration of the plurality of electrodes 421 of FIG. 4 may be identical in whole or part to the configuration of the plurality of electrodes 421 of FIG. 5, and the configuration of the rear cover 391 of FIG. 7 may be identical in whole or part to the configuration of the rear cover 391 of FIG. 4.

According to various embodiments, the rear cover 391 may form at least a portion of the outer surface of the electronic device 200. For example, the rear cover 391 may be a portion of a housing (e.g., the housing 210 of FIG. 2).

According to various embodiments, the rear cover 391 may include at least one transmission area 391a and 39b. For example, the transmission areas 391a and 391b may include a first transmission area 391b for transferring light to a light receiving unit (e.g., the light receiving unit 411 of FIG. 5) and a second transmission area 391b configured to allow at least a portion of the light radiated from the light emitting unit (e.g., the light emitting unit 413 of FIG. 5) to pass therethrough. According to various embodiments, the first transmission area 391a may surround at least a portion of the light receiving unit 411 or may face at least a portion of the light receiving unit 411, and the second transmission area 391b may surround at least a portion of the light emitting unit 413 or may face at least a portion of the light emitting unit 413. According to various embodiments, the transmission areas 391a and 391b may be through holes or openings formed in the rear cover 391. According to various embodiments, at least a portion of the rear cover 391 may be formed to be substantially transparent, and the transmission areas 391a and 391b may be interpreted as substantially transparent areas of the rear cover 391.

According to various embodiments, a plurality of electrodes 421 (e.g., the bio-impedance detection sensor 420 of FIG. 5) may be disposed on the rear cover 391. For example, the bio-impedance detection sensor 420 may include a first electrode 421a, a second electrode 421b, a third electrode 421c, and a fourth electrode 421d spaced apart from each other. According to various embodiments, some of the first electrode 421a, the second electrode 421b, the third electrode 421c, and the fourth electrode 421d may transmit a current, and other electrodes may receive a current. According to various embodiments, as the electronic device 200 uses the plurality of electrodes 421, the contact impedance due to the contact between the electrode 421 and the body may be reduced, and thus the accuracy of measuring the electrolyte concentration e may be increased. According to various embodiments, the plurality of electrodes 421 may be disposed adjacent to the transmission areas 391a and 391b.

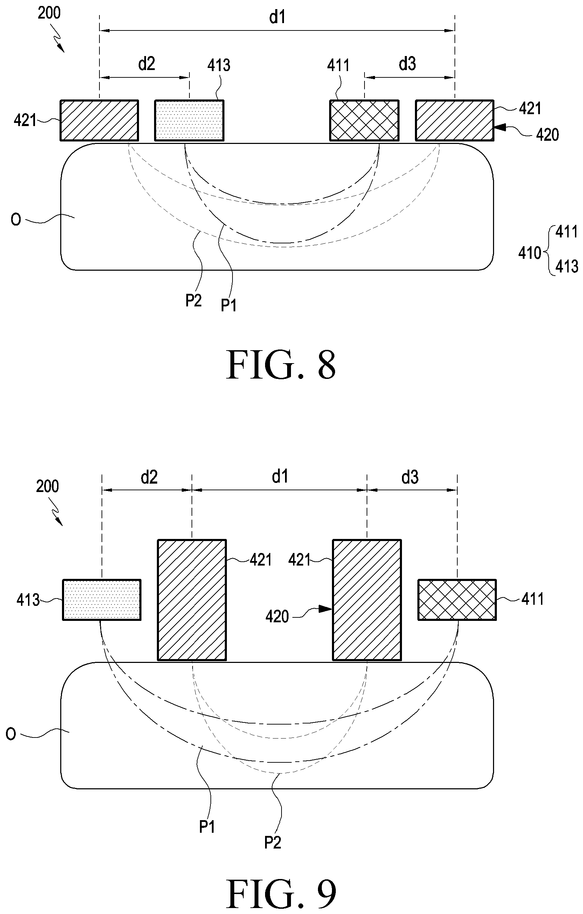

FIGS. 8 to 12 are views illustrating a placement structure of an electronic device including an optical sensor and a bio-impedance detection sensor according to various embodiments of the disclosure.

Referring to FIGS. 8 to 12, an electronic device 200 may include an optical sensor 410 and a bio-impedance detection sensor 420. The configuration of the optical sensor 410 and the bio-impedance detection sensor 420 of FIGS. 8 to 12 may be identical in whole or part to the configuration of the optical sensor 410 and the bio-impedance detection sensor 420 of FIG. 5.

According to various embodiments, the bio-impedance detection sensor 420 may be disposed adjacent to the optical sensor 410. As the optical sensor 410 is disposed adjacent to the biometric signal detection sensor 420, the accuracy of calculating the electrolyte concentration of the electronic device 200 may be enhanced. For example, as the volume in which the optical path P1 and the current path P2 overlap increases, the accuracy of determining the electrolyte concentration e may be enhanced. According to various embodiments, the ratio at which the optical path P1 and the current path P2 overlap may be referred to as a correlation of a path and, as the correlation is closer to 1, the accuracy of determining the electrolyte concentration e may increase. According to various embodiments, the correlation in which the optical path P1 and the current path P2 overlap may be changed based on a first distance d1 between the plurality of electrodes 421 (e.g., the light emitting unit and the light receiving unit), a second distance d2 between the light emitting unit 413 and the electrode 421, and a third distance d3 between the light receiving unit 411 and the electrode 421.

According to various embodiments, the correlation between the optical path P1 and the current path P2 measured by the electronic device 200 of the disclosure may be 0.7 or more. According to various embodiments, when the body is measured using the electronic device 200 in various body parts of the same person and the correlation is 0.7 or more, it may be determined that the electronic device 200 includes the optical sensor 410 and the bio-impedance detection sensor 420. The electronic device 200 may determine a biometric component (e.g., electrolyte concentration) based on the intensity of light detected using the optical sensor 410 and the magnitude of impedance detected using the bio-impedance detection sensor 420.

Referring to FIG. 8, the optical sensor 410 may be positioned between the bio-impedance detection sensors 420. For example, the light receiving unit 411 and the light emitting unit 413 may be positioned between the plurality of electrodes 421 of the bio-impedance detection sensor 420. According to various embodiments (e.g., FIG. 8), the first distance d1 may be about 18 mm, the second distance d2 may be about 2.5 mm to 4.5 mm, and the third distance d3 may be about 2.5 mm to 4.5 mm. For example, in the electronic device 200 having the first distance d1 of about 18 mm and the second distance d2 and the third distance d3 of about 3.5 mm, the correlation between the optical path P1 and the current path P2 may be 0.93. As another example, in the electronic device 200 in which the first distance d1 is about 18 mm, the second distance d2 is about 3.5 mm, and the third distance d3 is about 2.5 mm or about 4.5 mm, the correlation between the optical path P1 and the current path P2 may be 0.91 to 0.92.

Referring to FIG. 9, the bio-impedance detection sensor 420 may be positioned between the optical sensors 410. For example, the plurality of electrodes 421 of the bio-impedance detection sensor 420 may be positioned between the light receiving unit 411 and the light emitting unit 413. According to various embodiments (e.g., FIG. 9), the first distance d1 may be about 7 mm, the second distance d2 may be about 2.5 mm to 3.5 mm, and the third distance d3 may be about 2.5 mm to 3.5 mm. For example, in the electronic device 200 in which the first distance d1 is about 7 mm, the second distance d2 is about 2.5 mm, and the third distance d3 is about 2.5 mm or about 3.5 mm, the correlation between the optical path P1 and the current path P2 may be about 0.82.

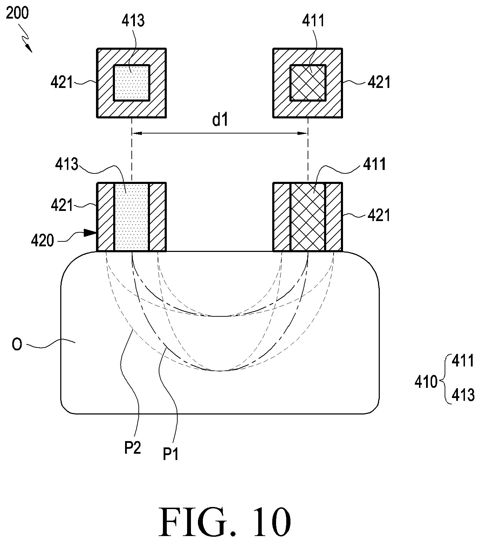

Referring to FIG. 10, the bio-impedance detection sensor 420 may surround at least a portion of the optical sensor 410. For example, some of the plurality of electrodes 421 may surround at least a portion of the light receiving unit 411, and other some of the plurality of electrodes 421 may surround at least a portion of the light emitting unit 413. According to various embodiments, the first distance d1 may be about 6.5 mm, and the correlation may be 0.76.

According to various embodiments, the electrode 421 of the bio-impedance detection sensor 420 may include a substantially transparent electrode. The electrode 421 may include indium tin oxide. As another example, if the transparent electrode 421 is provided, the material of the electrode 421 is not limited.

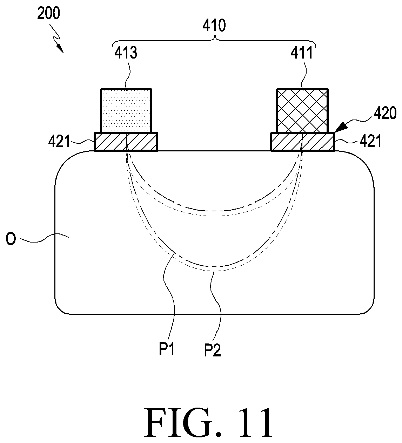

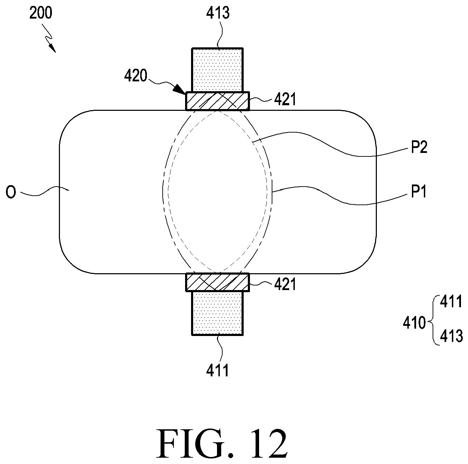

Referring to FIGS. 11 and 12, the optical sensor 410 may be disposed on the bio-impedance detection sensor 420. According to various embodiments, the light receiving unit 411 and the light emitting unit 413 may be disposed on different transparent electrodes 421, respectively. At least a portion of the light radiated from the light emitting unit 413 may be transferred to the user's body through the electrode 421 disposed under the light emitting unit 413. At least a portion of the light passing through the user's body may be transferred to the light receiving unit 411 through the electrode 421 disposed under the light receiving unit 411.

According to various embodiments, the electronic device 200 may detect the electrolyte concentration in various parts of the user's body. Referring to FIG. 11, the optical sensor 410 and the bio-impedance detection sensor 420 may be positioned substantially in one direction with respect to the user's body. According to various embodiments, the electronic device 200 may detect the electrolyte concentration in the user's wrist. Referring to FIG. 12, the light receiving unit 411 may be disposed to face at least a portion of the light emitting unit 413. For example, the light receiving unit 411 and the light emitting unit 413 may be disposed to accommodate the user's earlobe or the user's fingertip, and the electronic device 200 may detect the electrolyte concentration in the user's earlobe or the user's fingertip. According to various embodiments, the electronic device 200 of FIG. 12 may be referred to as a pinch type.

According to various embodiments of the disclosure, an electronic device (e.g., the electronic device 200 of FIG. 2) may comprise a housing (e.g., the housing 210 of FIG. 2), an optical sensor (e.g., the optical sensor 410 of FIG. 5) disposed on the housing and including a light receiving unit (e.g., the light receiving unit 411 of FIG. 5) and a light emitting unit (e.g., the light emitting unit 413 of FIG. 5) configured to radiate light to the light receiving unit through an optical path (e.g., the optical path P1 of FIG. 5), a bio-impedance detection sensor (e.g., the bio-impedance detection sensor 420 of FIG. 5) disposed adjacent to the optical sensor, and including a plurality of electrodes configured to transmit/receive a current through a current path (e.g., the current path P2 of FIG. 6), and a processor (e.g., the processor 120 of FIG. 1) disposed in the housing. At least a portion of the optical path may be configured to overlap at least a portion of the current path. The processor may be configured to determine an electrolyte concentration based on an intensity of light detected using the optical sensor and a magnitude of impedance sensed using the bio-impedance detection sensor.

According to various embodiments, the light receiving unit and the light emitting unit may be positioned between the plurality of electrodes.

According to various embodiments, the plurality of electrodes may be positioned between the light receiving unit and the light emitting unit.

According to various embodiments, the bio-impedance detection sensor may surround at least a portion of the optical sensor.

According to various embodiments, the plurality of electrodes may be substantially transparent electrodes. The light receiving unit and the light emitting unit may be disposed on the bio-impedance detection sensor.

According to various embodiments, the electronic device may further comprise a key input device (e.g., the key input device 203 or 204 of FIG. 2) disposed on the housing. The processor may be configured to determine at least one of an electrocardiogram or a body composition based on a user input transferred to the key input device.

According to various embodiments, the electronic device may further comprise a switching circuit (e.g., the switching circuit 330 of FIG. 6) configured to selectively and electrically connect the bio-impedance detection sensor or the key input device to the processor.

According to various embodiments, a wavelength range of light generated by the light emitting unit may be 1400 nm to 1500 nm or 1900 nm to 2000 nm.

According to various embodiments, a frequency of a current generated by the bio-impedance detection sensor may be 20 kHz or more.

The processor is configured to determine the electrolyte concentration using Equation 1 below: [Equation 1] electrolyte concentration $\left(e\right) = \frac{ε {\left(nd\right)}^{2}}{kZln \left(\frac{{I}_{0}}{I}\right)}$, where e denotes the electrolyte concentration, ε denotes a light absorption coefficient of an electrolyte in blood of an object (e.g., the object U of FIG. 5), d denotes a distance between the plurality of electrodes, n denotes a proportional constant between the current path and the electrode distance, k denotes a proportional constant between conductivity and the electrolyte concentration, Z denotes a magnitude of impedance detected by the bio-impedance detection sensor, I₀ denotes an intensity of light emitted from the light emitting unit, and I denotes an intensity of light received by the light receiving unit.

According to various embodiments, the electronic device further comprises a memory (e.g., the memory 130 of FIG. 1) configured to store the light absorption coefficient of the electrolyte, the proportional constant between the current path and the electrode distance, and the proportional constant between the conductivity and the electrolyte concentration.

The light absorption coefficient of the electrolyte, the proportional constant between the current path and the electrode distance, and the proportional constant between the conductivity and the electrolyte concentration are learned using machine learning.

According to various embodiments, the housing may include a rear cover (e.g., the rear cover 391 of FIG. 7) including a first transmission area (e.g., the first transmission area 391a of FIG. 7) facing at least a portion of the light receiving unit and a second transmission area (e.g., the second transmission area 391b of FIG. 7) facing at least a portion of the light emitting unit. The plurality of electrodes may be disposed on the rear cover.

According to various embodiments, the plurality of electrodes may include a first electrode (e.g., the first electrode 421a of FIG. 7), a second electrode (e.g., the second electrode 421b of FIG. 7), a third electrode (e.g., the third electrode 421c of FIG. 7), and a fourth electrode (e.g., the fourth electrode 421d of FIG. 7) that are spaced apart from each other.

According to various embodiments, the electronic device may further comprise at least one attachment member (e.g., the attachment member 250 or 260 of FIGS. 2 and 3) detachably connected to the housing.

According to various embodiments of the disclosure, an electronic device (e.g., the electronic device 200 of FIG. 2) may comprise a housing (e.g., the housing of FIG. 2), an optical sensor (e.g., the optical sensor 410 of FIG. 5) disposed on the housing and including a light emitting unit (e.g., the light emitting unit 413 of FIG. 5) configured to radiate light to an object (e.g., the object O of FIG. 5) and a light receiving unit (e.g., the light receiving unit 411 of FIG. 5) configured to detect an intensity of the light radiated from the light emitting unit, an bio-impedance detection sensor (e.g., the bio-impedance detection sensor 420 of FIG. 5) disposed adjacent to the optical sensor, and including a current transmitting unit (e.g., the electrode 421 of FIG. 5) configured to transmit a current and a current receiving unit (e.g., the electrode 421 of FIG. 5) configured to receive at least a portion of the current transmitted from the current transmitting unit, and a processor (e.g., the processor 120 of FIG. 1) disposed in the housing. The processor may be configured to determine an electrolyte concentration of the object based on an intensity of light detected using the optical sensor and a magnitude of impedance sensed using the bio-impedance detection sensor.

The processor is configured to determine the electrolyte concentration using Equation 1 below: [Equation 1] electrolyte concentration $\left(e\right) = \frac{ε {\left(nd\right)}^{2}}{kZln \left(\frac{{I}_{0}}{I}\right)}$, where e denotes the electrolyte concentration, ε denotes a light absorption coefficient of an electrolyte in a user's blood, d denotes a distance between the current transmitting unit and the current receiving unit, n denotes a proportional constant between the current path and the electrode distance, k denotes a proportional constant between conductivity and the electrolyte concentration, Z denotes a magnitude of impedance detected by the bio-impedance detection sensor, I₀ denotes an intensity of light emitted from the light emitting unit, and I denotes an intensity of light received by the light receiving unit.

The electronic device further comprises a memory (e.g., the memory 130 of FIG. 1) configured to store the light absorption coefficient of the electrolyte, the proportional constant between the current path and the electrode distance, the proportional constant between the conductivity and the electrolyte concentration, and the intensity of light emitted from the light emitting unit.

According to various embodiments, a wavelength range of light generated by the light emitting unit may be 1400 nm to 1500 nm or 1900 nm to 2000 nm.

According to various embodiments, at least a portion of the light emitted from the light emitting unit may be configured to be transferred to the light receiving unit along an optical path (e.g., the optical path P1 of FIG. 5), and at least a portion of the current transmitted from the current transmitting unit may be configured to be transferred to the current receiving unit along a current path (e.g., the current path P2 of FIG. 5). At least a portion of the optical path may overlap at least a portion of the current path.

It is apparent to one of ordinary skill in the art that the electronic device including the optical sensor and the bio-impedance detection sensor of the disclosure as described above are not limited to the above-described embodiments and those shown in the drawings, and various changes, modifications, or alterations may be made thereto.

## Claims

1. An electronic device (200), comprising:
a housing (210);
memory (130);
an optical sensor (410) disposed on the housing (210) and comprising a light receiving unit (411) and a light emitting unit (413) configured to radiate light to the light receiving unit (411) through an optical path (P1);
a bio-impedance detection sensor (420) disposed adjacent to the optical sensor (410), and comprising a plurality of electrodes (421), configured to transmit and receive a current through a current path (P2); and
one or more processor (120) disposed in the housing (210),
wherein at least a portion of the optical path (P1) overlaps at least a portion of the current path (P2), and
wherein the one or more processor (120) are configured to determine a biometric component based on an intensity of light detected by the optical sensor (410) and a magnitude of impedance sensed by the bio-impedance detection sensor (420),
wherein the biometric component comprises an electrolyte concentration, and
wherein the one or more processor (120) is configured to determine the electrolyte concentration using Equation 1 below: [Equation 1] $electrolyte concentration \left(e\right) = \frac{ϵ {\left(nd\right)}^{2}}{kZln \left(\frac{{I}_{0}}{I}\right)}$
wherein e denotes the electrolyte concentration, ε denotes a light absorption coefficient of an electrolyte in a user's blood, d denotes an electrode distance between the plurality of electrodes, n denotes a proportional constant between the current path and the electrode distance, k denotes a proportional constant between conductivity and the electrolyte concentration, Z denotes the magnitude of impedance detected by the bio-impedance detection sensor (420), I₀ denotes an intensity of light emitted from the light emitting unit (413), and I denotes an intensity of light received by the light receiving unit (411),
wherein the memory (130) is configured to store the light absorption coefficient of the electrolyte, the proportional constant between the current path and the electrode distance, the proportional constant between the conductivity and the electrolyte concentration, and the intensity of light emitted from the light emitting unit, and
wherein the light absorption coefficient of the electrolyte, the proportional constant between the current path (P2) and the electrode distance, the proportional constant between the conductivity and the electrolyte concentration are learned by training a model using machine learning on a plurality of clinical data and/or data of various users data obtained by applying the electronic device (200) including the optical sensor (410) and the bio-impedance detection sensor (420) to a plurality of subjects.

2. The electronic device (200) of claim 1, wherein the light receiving unit (411) and the light emitting unit (413) are between the plurality of electrodes (421).

3. The electronic device (200) of claim 1, wherein the plurality of electrodes (421) are between the light receiving unit (411) and the light emitting unit (413).

4. The electronic device (200) of claim 1, wherein the bio-impedance detection sensor (420) surrounds at least a portion of the optical sensor (410).

5. The electronic device (200) of claim 4, wherein the plurality of electrodes (421) are transparent electrodes, and wherein the light receiving unit (411) and the light emitting unit (413) are disposed on the bio-impedance detection sensor (420).

6. The electronic device (200) of claim 1, further comprising a key input device (202, 203, 204) disposed on the housing (210), and
wherein the one or more processor (120) is configured to determine at least one of an electrocardiogram or a body composition based on a user input provided to the key input device (202, 203, 204).

7. The electronic device (200) of claim 6, further comprising a switching circuit (430) configured to selectively and electrically connect the bio-impedance detection sensor (420) or the key input device (202, 203, 204) to the one or more processor (120).

8. The electronic device (200) of claim 1, wherein a wavelength range of the light generated by the light emitting unit (413) is 1400 nm to 1500 nm or 1900 nm to 2000 nm.

9. The electronic device (200) of claim 1, wherein a frequency of the current generated by the bio-impedance detection sensor (420) is 20 kHz or more.

10. The electronic device (200) of claim 1, wherein the housing (210) comprises a rear cover (391) comprising a first transmission area (391a) facing at least a portion of the light receiving unit (411) and a second transmission area (391b) facing at least a portion of the light emitting unit (413), and
wherein the plurality of electrodes (421) are disposed on the rear cover (391).

11. The electronic device (200) of claim 1, wherein at least a half of a volume of the optical path (P1) overlaps the current path (P2).

12. The electronic device (200) of claim 1, further comprising at least one attachment member (250, 260) detachably connected to the housing (210).

## Patentansprüche

1. Elektronische Vorrichtung (200), umfassend:
ein Gehäuse (210);
Speicher (130);
einen optischen Sensor (410), der an dem Gehäuse (210) angeordnet ist und eine Lichtempfangseinheit (411) und eine Lichtemissionseinheit (413) umfasst, die dazu konfiguriert ist, Licht durch einen optischen Pfad (P1) an die Lichtempfangseinheit (411) auszustrahlen;
einen Bioimpedanzdetektionssensor (420), der angrenzend an den optischen Sensor (410) angeordnet ist und eine Vielzahl von Elektroden (421) umfasst, die dazu konfiguriert sind, einen Strom durch einen Strompfad (P2) zu übertragen und zu empfangen; und
einen oder mehrere Prozessoren (120), die in dem Gehäuse (210) angeordnet sind,
wobei mindestens ein Abschnitt des optischen Pfads (P1) mindestens einen Abschnitt des Strompfads (P2) überlappt, und
wobei der eine oder die mehreren Prozessoren (120) dazu konfiguriert sind, eine biometrische Komponente basierend auf einer von dem optischen Sensor (410) detektierten Lichtintensität und einer von dem Bioimpedanzdetektionssensor (420) erfassten Impedanzgröße zu bestimmen,
wobei die biometrische Komponente eine Elektrolytkonzentration umfasst, und
wobei der eine oder die mehreren Prozessoren (120) dazu konfiguriert sind, die Elektrolytkonzentration unter Verwendung von Gleichung 1 unten zu bestimmen: [Gleichnung 1] $Elektrolytkonzentration \left(e\right) = \frac{ϵ {\left(nd\right)}^{2}}{kZln \left(\frac{{I}_{0}}{I}\right)}$
wobei e die Elektrolytkonzentration bezeichnet, ε einen Lichtabsorptionskoeffizienten eines Elektrolyten im Blut eines Benutzers bezeichnet, d einen Elektrodenabstand zwischen der Vielzahl von Elektroden bezeichnet, n eine proportionale Konstante zwischen dem Strompfad und dem Elektrodenabstand bezeichnet, k eine proportionale Konstante zwischen Leitfähigkeit und Elektrolytkonzentration bezeichnet, Z die von dem Bioimpedanzdetektionssensor (420) detektierte Impedanzgröße bezeichnet, I₀ eine von der Lichtemissionseinheit (413) emittierte Lichtintensität bezeichnet und I eine von der Lichtempfangseinheit (411) empfangene Lichtintensität bezeichnet,
wobei der Speicher (130) dazu konfiguriert ist, den Lichtabsorptionskoeffizienten des Elektrolyten, die proportionale Konstante zwischen dem Strompfad und dem Elektrodenabstand, die proportionale Konstante zwischen der Leitfähigkeit und der Elektrolytkonzentration und die Intensität des von der Lichtemissionseinheit emittierten Lichts zu speichern, und
wobei der Lichtabsorptionskoeffizient des Elektrolyten, die proportionale Konstante zwischen dem Strompfad (P2) und dem Elektrodenabstand, die proportionale Konstante zwischen der Leitfähigkeit und der Elektrolytkonzentration durch Trainieren eines Modells unter Verwendung von maschinellem Lernen an einer Vielzahl von klinischen Daten und/oder Daten verschiedener Benutzerdaten gelernt werden, die durch Anwenden der elektronischen Vorrichtung (200), die den optischen Sensor (410) und den Bioimpedanzdetektionssensor (420) beinhaltet, auf eine Vielzahl von Probanden erhalten werden.

2. Elektronische Vorrichtung (200) nach Anspruch 1, wobei sich die Lichtempfangseinheit (411) und die Lichtemissionseinheit (413) zwischen der Vielzahl von Elektroden (421) befinden.

3. Elektronische Vorrichtung (200) nach Anspruch 1, wobei sich die Vielzahl von Elektroden (421) zwischen der Lichtempfangseinheit (411) und der Lichtemissionseinheit (413) befindet.

4. Elektronische Vorrichtung (200) nach Anspruch 1, wobei der Bioimpedanzdetektionssensor (420) mindestens einen Abschnitt des optischen Sensors (410) umgibt.

5. Elektronische Vorrichtung (200) nach Anspruch 4, wobei die Vielzahl von Elektroden (421) transparente Elektroden sind, und
wobei die Lichtempfangseinheit (411) und die Lichtemissionseinheit (413) auf dem Bioimpedanzdetektionssensor (420) angeordnet sind.

6. Elektronische Vorrichtung (200) nach Anspruch 1, ferner umfassend eine Tasteneingabevorrichtung (202, 203, 204), die an dem Gehäuse (210) angeordnet ist, und
wobei der eine oder die mehreren Prozessoren (120) dazu konfiguriert sind, mindestens eines von einem Elektrokardiogramm oder einer Körperzusammensetzung basierend auf einer Benutzereingabe, die der Tasteneingabevorrichtung (202, 203, 204) bereitgestellt wird, zu bestimmen.

7. Elektronische Vorrichtung (200) nach Anspruch 6, ferner umfassend einen Schaltkreis (430), der dazu konfiguriert ist, den Bioimpedanzdetektionssensor (420) oder die Tasteneingabevorrichtung (202, 203, 204) selektiv und elektrisch mit dem einen oder den mehreren Prozessoren (120) zu verbinden.

8. Elektronische Vorrichtung (200) nach Anspruch 1, wobei ein Wellenlängenbereich des von der Lichtemissionseinheit (413) erzeugten Lichts 1400 nm bis 1500 nm oder 1900 nm bis 2000 nm beträgt.

9. Elektronische Vorrichtung (200) nach Anspruch 1, wobei eine Frequenz des durch den Bioimpedanzdetektionssensor (420) erzeugten Stroms 20 kHz oder mehr beträgt.

10. Elektronische Vorrichtung (200) nach Anspruch 1, wobei das Gehäuse (210) eine hintere Abdeckung (391) umfasst, die einen ersten Übertragungsbereich (391a), der mindestens einem Abschnitt der Lichtempfangseinheit (411) zugewandt ist, und einen zweiten Übertragungsbereich (391b), der mindestens einem Abschnitt der Lichtemissionseinheit (413) zugewandt ist, umfasst, und
wobei die Vielzahl von Elektroden (421) an der hinteren Abdeckung (391) angeordnet ist.

11. Elektronische Vorrichtung (200) nach Anspruch 1, wobei mindestens die Hälfte eines Volumens des optischen Pfads (P1) den Strompfad (P2) überlappt.

12. Elektronische Vorrichtung (200) nach Anspruch 1, ferner umfassend mindestens ein Befestigungselement (250, 260), das lösbar mit dem Gehäuse (210) verbunden ist.

## Revendications

1. Dispositif électronique (200), comprenant :
un boîtier (210) ;
une mémoire (130) ;
un capteur optique (410) disposé sur le boîtier (210) et comprenant une unité de réception de lumière (411) et une unité d'émission de lumière (413) configurée pour rayonner de la lumière vers l'unité de réception de lumière (411) à travers un chemin optique (P1) ;
un capteur de détection de bio-impédance (420) disposé adjacent au capteur optique (410) et comprenant une pluralité d'électrodes (421) configurées pour transmettre et recevoir un courant à travers un chemin de courant (P2) ; et
un ou plusieurs processeurs (120) disposés dans le boîtier (210),
au moins une partie du chemin optique (P1) chevauchant au moins une partie du chemin de courant (P2), et
ledit ou lesdits processeurs (120) étant configurés pour déterminer une composante biométrique sur la base d'une intensité de lumière détectée par le capteur optique (410) et d'une grandeur d'impédance détectée par le capteur de détection de bio-impédance (420),
ladite composante biométrique comprenant une concentration d'électrolyte, et
ledit ou lesdits processeurs (120) étant configurés pour déterminer la concentration en électrolytes au moyen de l'équation 1 ci-dessous : [Equation 1] $Concentration en électrolytes = \frac{ϵ {\left(nd\right)}^{2}}{kZln \left(\frac{{I}_{0}}{I}\right)}$
où e désigne la concentration en électrolyte, ε désigne un coefficient d'absorption de lumière d'un électrolyte dans le sang d'un utilisateur, d désigne une distance d'électrode entre la pluralité d'électrodes, n désigne une constante proportionnelle entre le chemin de courant et la distance d'électrode, k désigne une constante proportionnelle entre la conductivité et la concentration en électrolytes, Z désigne la grandeur d'impédance détectée par le capteur de détection de bio-impédance (420), I₀ désigne une intensité de lumière émise par l'unité d'émission de lumière (413), et I désigne une intensité de lumière reçue par l'unité de réception de lumière (411),
ladite mémoire (130) étant configurée pour stocker le coefficient d'absorption de lumière de l'électrolyte, la constante proportionnelle entre le chemin de courant et la distance d'électrode, la constante proportionnelle entre la conductivité et la concentration en électrolytes, et l'intensité de lumière émise par l'unité d'émission de lumière, et
ledit coefficient d'absorption de lumière de l'électrolyte, ladite constante proportionnelle entre le chemin de courant (P2) et la distance d'électrode, et ladite constante proportionnelle entre la conductivité et la concentration en électrolytes étant appris en formant un modèle au moyen d'un apprentissage automatique sur une pluralité de données cliniques et/ou de données de diverses données d'utilisateurs obtenues en appliquant le dispositif électronique (200) comprenant le capteur optique (410) et le capteur de détection de bio-impédance (420) sur une pluralité de sujets.

2. Dispositif électronique (200) selon la revendication 1, ladite unité de réception de lumière (411) et ladite unité d'émission de lumière (413) étant entre la pluralité d'électrodes (421).

3. Dispositif électronique (200) selon la revendication 1, ladite pluralité d'électrodes (421) étant entre l'unité de réception de lumière (411) et l'unité d'émission de lumière (413).

4. Dispositif électronique (200) selon la revendication 1, ledit capteur de détection de bio-impédance (420) entourant au moins une partie du capteur optique (410).

5. Dispositif électronique (200) selon la revendication 4, ladite pluralité d'électrodes (421) étant des électrodes transparentes, et
ladite unité de réception de lumière (411) et ladite unité d'émission de lumière (413) étant disposées sur le capteur de détection de bio-impédance (420).

6. Dispositif électronique (200) selon la revendication 1, comprenant en outre un dispositif d'entrée à touches (202, 203, 204) disposé sur le boîtier (210), et
ledit ou lesdits processeurs (120) étant configurés pour déterminer un électrocardiogramme et/ou une composition corporelle sur la base d'une entrée d'utilisateur fournie au dispositif d'entrée à touches (202, 203, 204).

7. Dispositif électronique (200) selon la revendication 6, comprenant en outre un circuit de commutation (430) configuré pour connecter de manière sélective et par voie électrique le capteur de détection de bio-impédance (420) ou le dispositif d'entrée à touches (202, 203, 204) au ou aux processeurs (120).

8. Dispositif électronique (200) selon la revendication 1, une plage de longueurs d'onde de la lumière générée par l'unité d'émission de lumière (413) étant comprise entre 1400 nm et 1500 nm ou entre 1900 nm et 2000 nm.

9. Dispositif électronique (200) selon la revendication 1, une fréquence du courant généré par le capteur de détection de bio-impédance (420) étant supérieure ou égale à 20 kHz.

10. Dispositif électronique (200) selon la revendication 1, ledit boîtier (210) comprenant un couvercle arrière (391) comprenant une première zone de transmission (391a) en regard d'au moins une partie de l'unité de réception de lumière (411) et une seconde zone de transmission (391b) en regard d'au moins une partie de l'unité d'émission de lumière (413), et
ladite pluralité d'électrodes (421) étant disposées sur le couvercle arrière (391).

11. Dispositif électronique (200) selon la revendication 1, au moins une moitié d'un volume du chemin optique (P1) chevauchant le chemin de courant (P2).

12. Dispositif électronique (200) selon la revendication 1, comprenant en outre au moins un élément de fixation (250, 260) relié amovible au boîtier (210).
